Europäisches Patentamt

European Patent Office

Office européen des brevets

(19)

(11) Publication number: **0 277 678**
**A1**

(12) **EUROPEAN PATENT APPLICATION**

(21) Application number: **88200076.3**

(22) Date of filing: **18.01.88**

(51) Int. Cl.⁴: **A61F 2/02**

(30) Priority: **19.01.87 NL 8700113**

(43) Date of publication of application:
**10.08.88 Bulletin 88/32**

(84) Designated Contracting States:
**AT BE CH DE ES FR GB GR IT LI LU NL SE**

(71) Applicant: **Stichting Science Park Groningen**
**Nijenborgh 16**
**NL-9747 AG Groningen(NL)**

(72) Inventor: **Nijenhuis, Atze Jan**
**Nieuwe Ebbingestraat 3 1A**
**NL-9712 NC Groningen(NL)**
Inventor: **Leenslag, Jan Willem**
**Beetstraat 10**
**BE-3055 Neerijse(BE)**
Inventor: **Pennings, Albertus Johannes**
**Ettenlaan 3**
**NL-9331 BE Norg(NL)**

(74) Representative: **Smulders, Theodorus A.H.J.**
**et al**
**Vereenigde Octrooibureaux Nieuwe Parklaan**
**107**
**NL-2587 BP 's-Gravenhage(NL)**

(54) **A graft suitable for treatment by reconstructive surgery and having tissue-specific porosity, and a process for making such graft.**

(57) This invention relates to a graft for use in reconstructive surgery to repair damaged tissue, such as bone tissue, cartilage tissue, vascular tissue or nerve tissue. According to the invention the graft is characterized by a porous matrix of an organic polymeric material having a bi-porous structure, i.e., having at least two different pore structures each having its own pore size and pore size distribution. The matrix is preferably biodegradable and preferably incorporates a fibrous and/or a granular material, preferably also biodegradable, and/or additives, such as growth factors. The invention also relates to a process for making bi-porous grafts.

# A graft suitable for treatment by reconstructive surgery and having tissue-specific porosity, and a process for making such graft.

This invention relates to a graft suitable for the treatment by reconstructive surgery of damaged cartilage tissue, bone tissue, vascular tissue and nerve-tissue.

It turns out that the healing process of damaged tissue can often be divided into several stages. In these, vascularization of the damaged tissue, in combination with the development of regenerative tissue appears to be a body reaction that is often essential.

When a graft is used for the treatment of damaged tissue, vascularization and the ingrowth of the several cell types into the graft can be greatly influenced by the pore size and pore size distribution in the graft used. Earlier studies have shown that the use of a graft with a pore size of about 400 μm results in a rapid ingrowth of the connective tissue. In the case of reconstructive bone surgery, the pore size considered to be optimal for the formation of bony tissue is 150 to 250 μm. Vascularization of a graft takes place especially when this graft contains pores of 10-60 μm. In the case of vascular prostheses, a pore size of 25 to 150 μm is used, with the pore size increasing from the inside outwardly.

An essential feature in all of the above grafts is the interconnectivity of the pores in three dimensions throughout the entire graft. The grafts must have a controlled and reproducible pore size and pore size distribution.

It is an object of the present invention to provide grafts of the above kind, made by a simple, reproducible, and generally applicable method.

To this effect, according to the invention, there is provided a graft of the above kind, characterized by a porous matrix of an organic polymeric material with a bi-porous structure.

The organic polymeric material of the porous matrix according to the invention is preferably biodegradable.

The porous matrix according to the invention may be compounded with a biodegradable fibrous material and/or with a biodegradable first granular material.

As a biodegradable organic polymeric matrix material for the graft according to the invention, use can be made of a polyurethane material, for example, a polyether urethane, a polyester urethane, a polyether urea urethane or a polyester urea urethane; a polylactide material, for example, a poly-L-lactide, a poly-D-lactide or a poly-DL-lactide; a polyglycolide material, for example, a polyglycol acid; a polylactone material, for example, a poly-δ-valerolactone, a poly-ε-caprolactone; a polyhydroxy-carboxylic acid material, for example, a poly-β-hydroxybutyric acid; a polyester material, for example, polytetramethylene adipate, polyethylene adipate, polyhexamethylene glutarate, polyethylene terephthalate; a hydroxy-carboxylic acid copolymeric material, for example, a lactide glycolide copolymer, a lactide-ε-caprolactone copolymer, a δ-valerolactone-ε-caprolactone copolymer, a β-hydroxy-octanoic acid-β-hydroxy-hexanoic acid copolymer. In the copolymers the pure L, D and DL stereoisomers of lactide can be used separately or as a mixture of these. Separate polymeric materials can be used or mixtures thereof, possibly with still other biodegradable organic polymeric materials, for example, with a polyamide material.

The fibrous material for reinforcement and/or as a stimulus for tissue ingrowth can be incorporated in the matrix in the form of loose fibres, but also in the form of a woven or knitted fabric or other coherent combination of fibres and comprises fibrous material of a biodegradable organic polymeric material, for example, fibres of a polyurethane material, for example, a polyether urethane, polyester urethane, polyether urea urethane, a polyester urea urethane; a polylactide material, for example, a poly-L-lactide, a poly-D-lactide, a poly-DL-lactide; a polyglycolide material, for example, a polyglycolic acid; a polylactone material, for example, a poly-ε-valerolactone, a poly-ε-caprolactone; a poly-hydroxycarboxylic acid material, for example, a poly-β-hy droxybutyric acid; a polyester material, for example, a polyethylene terephthalate, a polytetramethylene adipate, polyethylene adipate, polyhexamethylene glutarate; a hydroxycarboxylic acid copolymeric material, for example, a lactide glycolide copolymer, a lactide-ε-caprolactone copolymer and a δ-valerolactone-ε-caprolactone copolymer, a β-hydroxy-octanoic acid-β-hydroxy-hexanoic acid copolymer; a polyaramide material, for example, poly-p-aminobenzoic acid. Furthermore, it is possible to use fibres of a polyamide material, of collagenous material, or of polydioxanone. In the copolymers in which a lactide is used, it is possible to use the pure L, D and DL stereoisomers or mixtures of these. In producing the fibrous material, the starting material may be the separate polymeric materials specified hereinbefore, or mixtures thereof. Fibres of a single fibre material can be used or mixtures of fibres of different fibre materials. The fibres used may be solid, porous or hollow.

The first granular material for reinforcement and/or as a stimulus for tissue ingrowth of the composite according to the invention can be incorporated in the matrix in the form of loose granules. The granules to be used according to the invention are of a biodegradable, polymeric or non-polymeric, organic or inorganic material and comprise granules of the organic polymeric materials specified above or of inorganic salts, for example, tricalcium phosphate, calcium carbonate, calcium sulphate, magnesium phosphate. Granules of a single granule material can be used or granules of different granule materials. The granules used may be solid or porous.

To improve the adherence between fibres and/or granules and matrix, the fibres and/or granules can be pre-treated. This pre-treatment may comprise coating the fibres and/or granules with a suitable material or roughening and activating the fibre and/or granule surface by means of annealing.

In the graft according to the invention, additives can be incorporated to affect the biodegradability of the matrix and/or the biodegradability of the fibres and/or the biodegradability of the granulate. When a polyester urethane, a polyester urea urethane, a polyester or a poly(hydroxy-carboxylic acid) is used, these additives may consist of free carboxylic acids, amino acids or hydroxycarboxylic acids, for example, lactic acid, glycolic acid, citric acid, tartaric acid, glutaric acid, fumaric acid or salicylic acid. Other additives incorporated in the graft may be growth factors, for example FGF, CGF, NGF (Fibroblast Growth Factor, Chondrocyte Growth Factor, Nerve Growth Factor, respectively), growth promoting and growth inhibiting agents, antibiotics, sedatives, vitamins, building materials and nutrients.

Each of these additives or each preferred combination may be incorporated in the matrix material and/or fibrous material and/or first granulate material. Depending on the additive to be used and the purpose thereof, it may also be distributed over the pore walls of the graft, for example, by steeping the graft with a solution of the additive, and drying it. Hollow fibres closed at both ends and filled with one or more additives or a suspension or solution thereof can also be used.

The invention also relates to a process for making the graft and is characterized by subjecting a solidified polymeric solution under reduced pressure to a sublimation process to remove the solvent, the solidified polymeric solution being composed of the polymeric material or mixture of polymeric materials constituting the porous matrix; a sublimable solvent or a mixture of sublimable solvents, or a mixture of one or more sublimable solvents and one or more sublimable first non-solvents; and a second granular material different from said first granular material and capable of being washed out with a solvent which is not a solvent for the biodegradable organic polymeric matrix, referred to herein as the second non-solvent.

The solidified polymeric solution is built up from a minimum of three components. The first component consists of the polymer or the polymer mixture to form the matrix. The second component consists of a solvent or a solvent mixture or a mixture of one or more solvents with one or more first non-solvents. When the solution is cooled, depending on the solvents and first non-solvents used, phase separation by binodal or spinodal segregation, and/or eutectic crystallization will or will not occur, resulting in highly characteristic porous structures with a porosity that can be controlled by varying the concentration of the component materials. The third component comprises a second granular material which differs from said first granular material and comprises a ground, powdered or crystalline material having a pre-determinable particle size or particle size distribution. This second granular material may be of organic nature, for example, sugars, such as saccharose and lactose or, for example, urea, or it may be of inorganic nature, for example, an inorganic salt, such as NaCl, NaF, $Na_2SO_4$, KBr, or it may a mixture of organic and inorganic materials. The second granular material must be soluble in a solvent, the second non-solvent, which does not function as such for the polymeric matrix or, if used, the fibrous material and/or, if used, said first granulate. The object of the second granular material is to give a portion of the graft the desired porosity by washing out this material. A next component may be the above fibrous material and/or said first granular material and/or said additives.

A characteristic feature of the grafts according to the present invention is a bi-porous structure. This means that the graft comprises different pore structures, each having its own pore size and pore size distribution.

This structure is formed as a result of the fact that the pores caused by the second granular material are interconnected by a porous matrix. The relatively smaller pores in the matrix are caused by evaporating the polymer solvent or solvent mixture or a mixture of one or more solvents with one or more first non-solvents. The second granular material may have any selected particle size and particle size distribution. When a second granular material is used with a narrow particle size distribution, for example, 200 μm ± 10 μm, the pores formed by this material will be similar in size and size distribution. When a second granular material is used which has a plurality of different particle sizes, each with its own particle size distribution, for example, 100 μm ±10 μm, 150 μm ± 20 μm and 200 μm ± 15 μm, the pores formed by this material will, in turn, have a similar size and size distribution. In this case too, the structure is referred to as a bi-porous structure.

A graft according to the invention combines pores with pore sizes at no fewer than two different size levels. Without wishing to be limited by the following theory, we suppose that in all probability this promotes the simultaneous ingrowth of specific tissues, although it is not excluded that one tissue type exhibits a much faster ingrowth than the other.

A graft according to the invention with pores of 150-200 $\mu$m, formed by the second granular material, dispersed in a porous matrix having pores of 10-60 $\mu$m, formed by evaporation of the solvent, gives excellent results in reconstructive surgery of cartilaginous tissue. In the first instance vascularization of the graft takes place by capillary blood vessels which substantially grow through the microporous matrix. Cartilaginous cells are formed in the large pores in a later stage. When a graft according to the invention is used for reconstructive surgery of bony tissue, vascularization and regeneration of bony tissue take place in the graft at the same time.

When a graft according to the invention is used for the reconstruction of damaged nerve tissue, the graft, i.e., the nerve guide, serves no other function than stimulating the growing together of the separated nerve cell parts. The non-porous inside of the nerve guide is surrounded by a microporous outside. The ingrowth of surrounding tissue into the microporous outside provides for a good fixation of the nerve guide, whereby dislocation is prevented.

If the dimensional stability of a graft according to the invention for more prolonged periods of time is of importance, as may be the case with vascular prostheses, a very slowly biodegradable or non-biodegradable component may be incorporated in the graft. The retention of mechanical or physical properties may also give rise to such measures.

The invention is illustrated in and by the following examples of producing a graft.

## Example I

### A. The materials used for producing the composite in this example.

A commercial polyester urethane, sold under the tradename of Estane 5701-Fl (Goodrich Co., Brechville, Ohio, USA) was used. Furthermore, a poly-L-lactide was used with an $\overline{M}_v$ of up to $6.10^5$. From the poly-L-lactide, fibres were made by a well-known dry-spin-hot-stretch process. The poly-L-lactide used therefor had an $M_v$ of $5-6 \times 10^5$. The elongation at break of the fibres was 0.5-1.2 GPa and the Young's modulus was 6-15 GPa.

The poly-L-lactide used in the polymeric mixture had an $\overline{M}_v$ of $3.5.10^5$.

Crystalline saccharose (Merck) was sieved, and the fraction with a particle size of 100 - 300 $\mu$m was used.

### B. Preparing the polyurethane and the poly-L-lactide fibres.

The Estane 5701 Fl was precipitated once by precipitation of a 5% w/w polymer solution in dimethyl formamide (DMF) (Merck "pro-analysis" quality) in a six-fold volume of ice water. The precipitated polyurethane was washed once with 96% ethanol and once with diethyl ether, and was then dried overnight in a vacuum stove at 40°C. The poly-L-lactide fibres used were coated with Estane 5701-Fl by steeping them in a 5% w/w solution in tetrahydrofurane (THF) (Merck "pro analysis" grade) of the polyurethane, whereafter the fibres were dried in a vacuum desiccator for 1 hour.

### C. Preparing the porous composite.

0.1 g poly-L-lactide ($\overline{M}_v$ $3.5.10^5$) was dissolved in 8 g 1,4-dioxane (Merck "pro-analysis" quality) at 55°C. Subsequently, 1.9 g Estane 5701-Fl was added to this solution and dissolved. The solution, which was slightly turbid owing to phase segregation of the polymer-polymer solvent system, was mixed with 5 g sugar crystals (grain size 100-300 $\mu$m) and 0.4 g poly-L-lactide fibres, the latter being cut to pieces of 3-5 mm. The homogeneous mixture was, after mixing, rapidly frozen uniaxially in liquid nitrogen, and then kept in the liquid nitrogen for another 5 minutes to permit effective cooling. The resulting disk, with a height of about 1 cm and a diameter of about 4 cm, was dried at 0.005 mbar for 12 hours to remove the 1,4-dioxane. The resulting porous sample was extracted with water for 24 hours to remove the sugar crystals. A last extraction with 96% ethanol was effected for 24 hours to remove residues of 1,4-dioxane. After drying the sponge was porous as to about 65% and contained 17% poly-L-lactide fibres. Pores with a size of 80-300 $\mu$m, formed by the sugar crystals used, turned out to be interconnected by channel-shaped pores with a diameter of about 10 $\mu$m, caused by the evaporation of the 1,4-dioxane.

## Example II

A. The materials used for producing the composite in this example.

Polyurethane and poly-L-lactide were used as in Example I.

Sodium chloride crystals (Merck "pro-analysis" quality) were sieved, and the fraction with a particle size of 100-300 µm was used.

B. Preparing the polyurethane used.

This was carried out as in Example 1.

C. Preparing the porous composite.

0.5 g poly-L-lactide with an $\overline{M}_v$ of $3.5.10^5$ was dissolved in a mixture of 20 g trioxane (Merck "pro-analysis" quality) and 20 g 1,4-dioxane. Subsequently, 9.5 g of Estane 5701-FI was added to this solution and dissolved. The solution, which was slightly turbid from phase segregation of the polymer-polymer solvent system, was mixed with 42 g NaCl granules with a particle size of 100-300 µm. A glass dish with a diameter of 4 cm and a height of 1 cm was filled with a portion of the mixture, whereafter the mixture was frozen at -15°C. The sample was kept at -15°C for 12 hours, whereafter it was dried at 0.01 mbar for 12 hours. Thereafter the porous material was extracted with 96% ethanol for 24 hours to remove the last residues of trioxane and 1,4-dioxane. To remove the sodium chloride granules, it was subsequently extracted with water for 24 hours. After drying the sponge was porous as to about 70-75%. Pores with a size of 80-300 µm, formed by the salt crystals used, were found to be interconnected by pores with a size of 10-60 µm, formed by evaporating the solvent crystallized. The size of the pores caused by the evaporation of the solvent could be greatly influenced by the ratio of trioxane : dioxane. When a 70% trioxane/30% dioxane mixture is used, these pores reach a size of 20-150 µm.

Example III

A. The materials used to produce the composite in this example.

These were as in Example I.

B. The preparation of the polyurethane used.

This was effected as in Example I.

C. Producing the porous composite.

To a solution of 3.3 g Estane 5701-FI in 13.3 g 1,4-dioxane, 8 g 1,4-butanediol (Merck "pro-analysis" quality) was slowly added at 65°C. This solution was mixed with 20 g sodium chloride crystals having a diameter of 100-300 µm. The resulting paste was poured to form a disk having a diameter of about 10 cm and a height of several millimeters. The sample was cooled at -10°C for 12 hours, whereafter it was dried at 0.01 mbar for 12 hours. To remove the last residues of 1,4-dioxane and 1,4-butanediol, it was extracted in 96% ethanol for 24 hours. To remove the sodium chloride crystals and the last residues of 1,4-butanediol, the sample was extracted in water for 62 hours.

Pores with a size of 80-300 µm, formed by the salt used, were found to be interconnected by a highly porous matrix having pores of 5-100 µm. These pores have been formed by phase separation in the cooling solution of the polymer solvent/non-solvent system, followed by drying at reduced pressure of the solidified solution. It was possible for the pore size in the matrix to be greatly affected by the rate at which the solution was cooled.

To produce a graft according to the invention in a specific form, for example, a meniscus form or a blood vessel form, a mould can be used. The solidified polymer solution, built up from the above components, is removed from the mould and then prepared further in accordance with the process. From the graft material, grafts can also be made to size by hand or machining. If necessary the graft material may be cooled to improve its machinability. To produce tubular grafts, one or more layers of a polymer solution, built up from the various components specified hereinbefore, can be applied to a tube or rod which, if necessary, is cooled. The tube or rod with the solidified polymer solution thereon can then be prepared further in accordance with the process. When more layers of a polymer solution are used, this solution may be varied in composition layer by layer, so that the porosity, biodegradability and physical properties can be controlled for each layer separately.

**Claims**

1. A graft suitable for the treatment by reconstructive surgery of damaged bone tissue, cartilage tissue, vascular tissue and nerve-tissue, characterized by a porous matrix of an organic polymeric material with a bi-porous structure.

2. A graft as claimed in claim 1, characterized in that the organic polymeric material is biodegradable.

3. A graft as claimed in claim 1 or 2, characterized in that the matrix incorporates a biodegradable organic polymeric fibrous material in the form of loose fibres and/or in the form of a coherent fibre combination and/or a biodegradable first granular material.

4. A graft as claimed in any of claims 1-3, characterized in that the matrix material and/or fibre material and/or the first granular material incorporates one or more growth factors or growth promoting agents, and/or that the growth factors and/or growth promoting agents are distributed over the pore walls of the graft.

5. A graft as claimed in claim 1 or 3, characterized in that one of the components is a very slowly biodegradable or non-biodegradable component.

6. A graft as claimed in claim 1, characterized in that the bi-porous structure comprises pores having a pore size of 80-300 $\mu$m, dispersed in the porous matrix having pores of a size of 5-100 $\mu$m.

7. A graft as claimed in claim 6, characterized in that the bi-porous structure comprises pores having a pore size of 150-250 $\mu$m, dispersed in the porous matrix having pores of a size of 10-60 $\mu$m.

8. A process for making a graft as claimed in any of claims 1-7, characterized by subjecting a solidified polymeric solution under reduced pressure to a sublimation process to remove the solvent, the solidified polymeric solution being composed of the polymeric material or mixture of polymeric materials constituting the porous matrix; a sublimable solvent or a mixture of sublimable solvents or a mixture of one or more sublimable solvents and one or more sublimable first non-solvents; and a second granular material different from said first granular material and capable of being washed out with a solvent which is not a solvent for the biodegradable organic polymeric matrix.

9. A process as claimed in claim 8, characterized in that during the solidification of the polymeric solution crystallization or eutectic crystallization takes place.

10. A process as claimed in claim 8 which includes the use of a first non-solvent, characterized in that during the solidification of the polymeric solution phase separation takes place.

11. A process as claimed in claim 8, characterized in that the solidified polymer solution includes as components the biodegradable organic polymeric fibrous material in the form of loose fibres and/or in the form of a coherent fibre combination and/or a biodegradable first granular material, which components are insoluble in the solvent for the second granular material which is a non-solvent for the biodegradable organic polymeric matrix.

12. A process as claimed in claim 11, characterized in that the second granular material is a ground, powdered or crystalline material having a pre-determinable particle size or particle size distribution.

13. A process as claimed in claim 12, characterized in that the second granular material is of organic and/or inorganic nature.

14. A process as claimed in claim 13, characterized in that the second granular material is a sugar.

15. A process as claimed in claim 13, characterized in that the second granular material is urea.

16. A process as claimed in claim 13, characterized in that the second granular material is an inorganic salt and/or organic salt.

## DOCUMENTS CONSIDERED TO BE RELÉVANT

| Category | Citation of document with indication, where appropriate, of relevant passages | Relevant to claim | CLASSIFICATION OF THE APPLICATION (Int. Cl. 4) |
|---|---|---|---|
| A | US-A-4 553 272  (MEARS)<br>* Abstract; figures; column 4, lines 28-45 * | 1,6,7 | A 61 F    2/02 |
| A | US-A-4 355 426  (McGREGOR)<br>* Claim 1 * | 1 | |
| A | FR-A-2 548 661  (SUMITOMO CEMENT) | | |
| A | US-A-3 890 107  (WHITE) | | |

TECHNICAL FIELDS SEARCHED (Int. Cl.4)

A 61 F
A 61 L

The present search report has been drawn up for all claims

| Place of search | Date of completion of the search | Examiner |
|---|---|---|
| THE HAGUE | 25-04-1988 | STEENBAKKER J. |